(19) Europäisches Patentamt
European Patent Office
Office européen des brevets

(11) EP 1 692 084 B1

# (12) EUROPEAN PATENT SPECIFICATION

(45) Date of publication and mention of the grant of the patent:
**09.05.2007 Bulletin 2007/19**

(21) Application number: **04803202.3**

(22) Date of filing: **20.11.2004**

(51) Int Cl.:
***C07B 57/00*** *(2006.01)* ***C12P 13/04*** *(2006.01)*
***C07C 227/34*** *(2006.01)*

(86) International application number:
**PCT/EP2004/013194**

(87) International publication number:
**WO 2005/058776 (30.06.2005 Gazette 2005/26)**

## (54) PROCESS FOR THE PREPARATION OF ENANTIOMERICALLY ENRICHED AMINO ACIDS

VERFAHREN ZUR HERSTELLUNG VON ENANTIOMEREN ANGEREICHERTEN AMINOSÄUREN

PROCEDE DE PREPARATION D'ACIDES AMINES ENRICHIS ENANTIOMERIQUEMENT

(84) Designated Contracting States:
**AT BE BG CH CY CZ DE DK EE ES FI FR GB GR HU IE IS IT LI LU MC NL PL PT RO SE SI SK TR**

(30) Priority: **10.12.2003 EP 03028341**

(43) Date of publication of application:
**23.08.2006 Bulletin 2006/34**

(73) Proprietor: **Degussa GmbH**
**40474 Düsseldorf (DE)**

(72) Inventors:
- **HOFFMANN, Rolf**
  **63546 Hammersbach (DE)**
- **KRAFT, Michael**
  **63517 Rodenbach (DE)**

(56) References cited:
**WO-A-03/016245** **DE-A1- 10 220 740**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 692 084 B1

## Description

The present invention is concerned with a process for the resolution of a mixture of enantiomers of N-protected amino acids, in particular $\alpha$-amino acids. Especially, this process claims a crystallisation of a diastereomeric salt pair consisting of one enantiomer of an N-protected amino acid and an enantiomerically pure N-unprotected $\beta$-amino acid derivative.

Enantiomerically enriched amino acids, especially $\alpha$-amino acids, are versatile tools for synthesising enantiomerically pure bio-actives, intermediates for pharmaceuticals or for infusion liquors for parenteral nutrition. A large amount of these amino acids, especially L-lysine, is produced for feed additive compositions.

The resolution of mixtures of enantiomers of amino acids, e.g. racemates, via classical crystallisation techniques is a rather old and common method for the generation of enantiomerically enriched amino acids. Although there have been great efforts to create new methodologies which allow to synthesise the amino acids in pure form via e.g. biotechnological means, the classical pathway is still an option for special amino acids which are not or only poorly generated by these methods (Izumi et al. Angew. chem. 1978, 90, 187; B. Hoppe et al. Chemie in unserer Zeit, 1984, 18, 73).

The object of the present invention was, therefore, to create a technical process for the resolution of enantiomeric mixtures of such amino acids. In particular the process should allow to gain especially pure forms of one enantiomer of these amino acids in a robust and easy to handle mode. From the perspective of an economical and ecological viewpoint the present process shall be advantageous over those described in the prior art, in particular for the generation of those amino acids which are disadvantageously produced via known methods.

Mentioned objective is addressed properly by applying a process according to features of present claim 1 or 7. Independent claims 2 to 6 or 8 to 11depict preferred embodiments of the process according to the invention.

By performing a process for the preparation of enantiomerically enriched N-protected amino acids, wherein a mixture of both enantiomers of an N-protected amino acid is resolved through crystallisation of the salt of one enantiomer of the N-protected amino acid and an enantiomerically pure N-unprotected $\beta$-amino acid derivative, the solution to the mentioned object is attained, in particular in a manner that is surprising, by no means foreseeable and, according to the invention, particularly advantageous. The instant process is very feasibly performed even on large scale and allows to produce enantiomerically enriched amino acids in an extremely easy and robust fashion. Nonetheless, the results achieved in view of enantiomeric purity and yield are outstandingly superb in the light of the easiness of the process of the invention.

In view of the N-protected amino acids used for instant process, these can be chosen by the skilled man. Amino acids preferably resolved are, however, $\alpha$- and $\beta$-amino acids. Taking these N-protected $\alpha$-amino acids are most preferred.

The process of the present invention is advantageously performed in an organic solvent which can contain water to an extent that it does not adversely affect the crystallisation, e. g. through phase separation. In particular the solvent used is a polar one, preferably selected from the group consisting of esters, ethers, ketones, alkohols, aromatic hydrocarbons or mixtures thereof. Especially preferred is a process in which the solvent is selected from the group consisting of ethyl acetate, ethanol, methyl-tert.-butyl ether, toluene.

The choice of an enantiomerically pure N-unprotected $\beta$-amino acid derivative as resolving agent is up to the skilled man's discretion. The $\beta$-amino acid derivative used may be chosen by performing routine testing in present process and comparing the results. Enaniomerically pure $\beta$-amino acids are commercially available or can advantageously be made according to processes known in the art (DE10220740 or DE10320211 and cited literature). Also the generation of the respective derivatives, like esters or amides, is known to the skilled worker (Chemistry of Amino Acids, Wiley&Sons 1961, J.P. Greenstein, M Winitz). Preferably an enantiomerically pure N-unprotected $\beta$-amino acid derivative according to formula (I) or (II) is used,

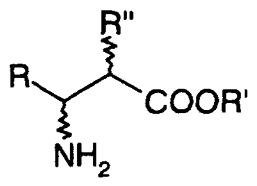 (I) 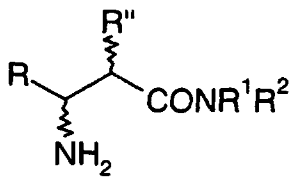 (II)

wherein

$R^1$, $R^2$ independently of one another denote H or R, R, R' independently of one another denote $(C_1\text{-}C_8)$-alkyl,

$(C_2-C_8)$-alkoxyalkyl, $(C_6-C_{18})$-aryl, $(C_7-C_{19})$ -aralkyl, $(C_3-C_{18})$-heteroaryl, $(C_4-C_{19})$-heteroaralkyl, $(C_1-C_8)$-alkyl-$(C_6-C_{18})$-aryl, $(C_1-C_8)$-alkyl-$(C_3-C_{18})$-heteroaryl, $(C_3-C_8)$-cycloalkyl, $(C_1-C_8)$-alkyl-$(C_3-C_8)$-cycloalkyl, $(C_3-C_8)$-cycloalkyl-$(C_1-C_8)$-alkyl,

or R and R" or R and R'/$R^1$ or R" and R'/$R^1$ represent a $(C_3-C_5)$-alkylene bridge mono- or polysubstituted with $(C_1-C_8)$-alkyl, HO- $(C_1-C_8)$ -alkyl, $(C_1-C_8)$-alkoxy, $(C_2-C_8)$-alkoxyalkyl, $(C_6-C_{18})$-aryl, $(C_7-C_{19})$-aralkyl, $(C_1-C_8)$-alkyl-$(C_6-C_{18})$-aryl, $(C_3-C_8)$-cycloalkyl, $(C_1-C_8)$-alkyl-$(C_3-C_8)$-cycloalkyl, $(C_3-C_8)$-cycloalkyl-$(C_1-C_8)$-alkyl,

R" being HO-, $(C_1-C_8)$-alkyl, $(C_2-C_8)$-alkoxyalkyl, $(C_6-C_{18})$-aryl, $(C_7-C_{19})$-aralkyl, $(C_3-C_{18})$-heteroaryl, $(C_4-C_{19})$-heteroaralkyl, $(C_1-C_8)$-alkyl-$(C_6-C_{18})$-aryl, $(C_1-C_8)$-alkyl-$(C_3-C_{18})$-heteroaryl, $(C_3-C_8)$-cycloalkyl, $(C_1-C_8)$-alkyl-$(C_3-C_8)$-cycloalkyl, $(C_3-C_8)$-cycloalkyl- $(C_1-C_8)$-alkyl.

Especially, suitable for present process are esters of β-amino acids. Preferably aromatic ester like 3-amino-3-phenylpropionic acid ethyl ester or the respective amide is used.

The temperature applied during the crystallisation process shall be as high or as low to allow a maximum crystallisation yield with an optimum of enantiomeric excess in view of the N-protected amino acids. The temperature is, therefore, preferably held within a range of -30° to 100°C. More preferably the temperature is set to -25 to 50°C, and most preferred the temperature varies between -20°C and 30°C.

After crystallisation the mixture is worked-up according to the knowledge of a skilled worker. Most convenient seems to be to separate the solid material from the mixture via filtration. Subsequently the already highly enriched diastereomeric salt pair can be recrystallised to maximise diastereomeric purity. Afterwards the enantiomerically enriched N-protected amino acid can be liberated from this salt by measures known to the man in the art, e.g. ion-exchange or classical acidification and extraction techniques.

It is possible in view of the instant invention to exchange substrates and crystallising agent for each other to make the process one, which allows to obtain enantiomerically enriched N-unprotected β-amino aicds. Hence, the present invention also concerns a process for the preparation of enantiomerically enriched N-unprotected β-amino acid derivates, wherein a mixture of both enantimers of an N-unprotected β-amino acid derivative is resolved through crystallisation of the salt of one enantiomer of the N-unprotected β-amino acid derivative and an enantiomerically pure N-protected α-amino acid. Preferred embodiments of this process are in line with those mentioned for performing the process for the preparation of N-protected amino acids.

Thus the present invention can easily be performed e.g. by dissolving the racemic N-protected amino acid in an appropriate solvent. Upon total dissolution the enantiomerically pure N-unprotected β-amino acid derivative, especially the ester, is added in an amount sufficient to obtain optimal crystallisation results in view of yield and diastereomeric purity. The molar ratio of added enantiomerically pure N-unprotected β-amino acid derivative to the antipode of N-protected amino acid to be crystallised can vary from 1:0,1 to 1:2, preferably from 1:0,5 to 1:1,8 or most preferably from 1:0,7 to 1:1,5. Preferably after cooling and completion of crystallisation the solid material is - as already stated - separated via filtration from the mixture and optionally recrystallised. Liberation of the desired N-protected amino acid takes place preferably through acidification and extraction.

In view of the production of β-amino acid derivates above mentioned procedure can be applied like with the exception that N-protected amino acids have to be substituted for N-unprotected β-amino acid derivates and vice versa.

N-Protected amino acid mean any carboxylic acid having both a carboxyl-function and an protected amino-function within one molecule. Preferably an α-amino acid being protected by a common protective group at the α-nitrogen atom of the amino acid is used. Suitable protective groups are known to the skilled worker (Green et al. Protective Groups in Organic Chemistry, Wiley&Sons, 1981). Preferably residues selected from the groups consisting of Z-, Boc-, Moc-, Eoc-, Fmoc-, formyl-, acetyl-, phthaloyl-radicals are chosen.

α-Amino acid denotes a naturally or unnaturally occurring amino acid like depicted in Bayer-Walter, Lehrbuch der organischen Chemie, S. Hirzel Verlag, Stuttgart, 22. Auflage, S. 822 et seq. Unnatural amino acids are those mentioned for example in DE19903268.8. Most preferred in this regard is the resolution of N-protected tert.-leucine.

Methyl, ethyl, *n*-propyl, isopropyl, *n*-butyl, isobutyl, sec-butyl, *tert*-butyl, pentyl, hexyl, heptyl or octyl, together with all their bond isomers, can be considered as $(C_1-C_8)$-alkyl radicals.

The $(C_1-C_8)$-alkoxy radical corresponds to the $(C_1-C_8)$-alkyl radical, with the proviso that this is bonded to the molecule via an oxygen atom.

As $(C_2-C_8)$-alkoxyalkyl, radicals in which the alkyl chain is interrupted by at least one oxygen function are meant, wherein two oxygen atoms cannot be joined to one another. The number of carbon atoms gives the total number of carbon atoms contained in the radical.

A $(C_3-C_5)$-alkylene bridge is a carbon chain with three to five C atoms, this chain being bonded to the molecule in question via two different C atoms. The bridge optionally can be unsaturated and or can contain one or more heteroatoms like N, O, S within the chain.

The radicals just described can be mono- or polysubstituted with $(C_1-C_8)$-alkoxy, $(C_1-C_8)$-alkyl, halogens and/or radicals containing N, O, P, S or Si atoms. These are particularly alkyl radicals of the type mentioned above having one or more

of these heteroatoms in their chain or being bonded to the molecule via one of these heteroatoms.

($C_3$-$C_8$) -Cycloalkyl means cyclopropyl, cyclobutyl, cyclopentyl, cyclohexyl or cycloheptyl radicals etc. These can be substituted with one or more halogens and/or radicals containing N, O, P, S or Si atoms and/or can have N, O, P or S atoms in the ring, such as e.g. 1-, 2-, 3-, 4-piperidyl, 1-, 2-, 3-pyrrolidinyl, 2-, 3-tetrahydrofuryl, 2-, 3-, 4-morpholinyl.

A ($C_3$-$C_8$)-Cycloalkyl-($C_1$-$C_8$)-alkyl radical refers to a cycloalkyl radical as set out above, which is bonded to the molecule via an alkyl radical as stated above.

($C_1$-$C_8$)-Acyloxy within the framework of the invention means an alkyl radical as defined above with a maximum of 8 C atoms, which is bonded to the molecule via a COO- function.

($C_1$-$C_8$)-Acyl within the framework of the invention means an alkyl radical as defined above with a maximum of 8 C atoms, which is bonded to the molecule via a CO- function.

A ($C_6$-$C_{18}$)-aryl radical is understood to mean an aromatic radical with 6 to 18 C atoms. These include in particular compounds such as phenyl, naphthyl, anthryl, phenanthryl or biphenyl radicals, or systems of the type described above annelated to the molecule in question, such as e.g. indenyl systems, which can optionally be substituted with ($C_1$-$C_8$)-alkyl, ($C_1$-$C_8$)-alkoxy, ($C_1$-$C_8$)-acyl or ($C_1$-$C_8$)-acyloxy.

A ($C_7$-$C_{19}$)-aralkyl radical is a ($C_6$-$C_{18}$)-aryl radical bonded to the molecule via a ($C_1$-$C_8$)-alkyl radical.

A ($C_3$-$C_{18}$)-heteroaryl radical within the framework of the invention refers to a five-, six- or seven-membered aromatic ring system of 3 to 18 C atoms, which contains heteroatoms such as e.g. nitrogen, oxygen or sulfur in the ring. In particular, radicals such as 1-, 2-, 3-furyl, such as 1-, 2-, 3-pyrrolyl, 1-, 2-, 3-thienyl, 2-, 3-, 4-pyridyl, 2-, 3-, 4-, 5-, 6-, 7-indolyl, 3-, 4-, 5-pyrazolyl, 2-, 4-, 5-imidazolyl, acridinyl, quinolinyl, phenanthridinyl and 2-, 4-, 5-, 6-pyrimidinyl are considered as such heteroaromatics.

A ($C_4$-$C_{19}$)-heteroaralkyl means a heteroaromatic system corresponding to the ($C_7$-$C_{19}$)-aralkyl radical.

Fluorine, chlorine, bromine and iodine are suitable as halogens (Hal).

The term enantiomerically enriched within the framework of the invention means the proportion of an enantiomer in a mixture with its optical antipode in a range of >50 % and <100 %. The ee (enantiomeric excess) value is calculated as follows:

$$([\text{Enantiomer1}]-[\text{Enantiomer2}])/([\text{Enantiomer1}]+[\text{Enantiomer2}])=\text{ee value}$$

The term diastereomerically enriched or diastereomeric purity is meant as to define the proportion of one diastereomer in a mixture with its other diastereomers in a range of >50 % and <100 %.

The naming of the molecules used according to the invention contains, within the framework of the invention, all possible diastereomers, the two optical antipodes of any diastereomer also being included therein.

Examples:

Example 1:

A solution of 59,9 g Benzyloxycarbonylphenylalanine in ethyl acetate was treated with 60 g (R)-3-Amino-3-phenylpropionic acid ethyl ester in ethyl acetate at 35°C, cooled to 0°C and stirred for 40 h. After filtration and drying 30,5 g of a 1:1 salt of R-Benzyloxycarbonylphenylalanine x (R)-3-Amino-3-phenylpropionic acid ethyl ester (86:14 optical purity) was obtained.

Example 2:

A solution of 50,2 g Benzyloxycarbonylvaline in methyl-tert.-butyl ether was treated with 60 g (S)-3-Amino-3-phenylpropionic acid ethyl ester in methyl-tert.-butyl ether at 30°C, cooled to 0°C and stirred for 30 h. After filtration and drying 27,1 g of a 1:1 salt of (S)-Benzyloxycarbonylvaline x (S)-3-Amino-3-phenylpropionic acid ethyl ester (97:3 optical purity) was obtained.

Example 3:

A solution of 47,6 g Benzyloxycarbonylamino-butyric acid in methyl-tert.-butyl ether was treated with 21,3 g (S)-3-Amino-3-phenylpropionic acid ethyl ester in methyl-tert.-butyl ether at 30°C, cooled to 10°C and stirred for 10 h. After filtration and drying 45,1 g of a 1:1 salt of (S)-Benzyloxycarbonylamino-butyric acid x (S)-3-Amino-3-phenylpropionic acid ethyl ester (92:8 optical purity) was obtained.

Example 4:

A solution of 106,1 g Benzyloxycarbonylamino-tert.leucine in methyl-tert.-butyl ether was treated with 42,56 g (R)-3-Amino-3-phenylpropionic acid ethyl ester in methyl-tert.-butyl ether at 35°C, cooled to 0°C and stirred for 15 h. After filtration and drying 71,8 g of a 1:1 salt of (R)-Benzyloxycarbonylamino-butyric acid x (R)-3-Amino-3-phenylpropionic acid ethyl ester (97:3 optical purity) was obtained.

Example 5:

A solution of 59,9 g Benzyloxycarbonylamino-tert.-leucine in ethyl acetate was treated with 60 g (R)-3-Amino-3-phenylpropionic acid ethyl ester in ethyl acetate at 42°C, cooled to -10°C and stirred for 10 h. After filtration and drying 38,5 g of a 1:1 salt of (R)-Benzyloxycarbonylamino-tert.-leucine x (R)-3-Amino-3-phenylpropionic acid ethyl ester (98,8: 1,2 optical purity) was obtained.

After recristallisation all salt pairs were obtained in an optical purity of >99:1.

## Claims

1. Process for the preparation of enantiomerically enriched N-protected amino acids, wherein a mixture of both enantiomers of an N-protected amino acid is resolved through crystallisation of the salt of one enantiomer of the N-protected amino acid and an enantiomerically pure N-unprotected β-amino acid derivative.

2. Process according to claim 1, wherein the N-protected amino acid is an N-protected α-amino acid.

3. Process according to claim 1 and/or 2, wherein the crystallisation is performed in a polar solvent.

4. Process according to one or more of the preceding claims, wherein the enantiomerically pure N-unprotected β-amino acid derivative is an ester or an amide.

5. Process according to one or more of the preceding claims, wherein the temperature during crystallisation is between -20°C and 30°C.

6. Process according to one or more of the preceding claims, wherein the crystallised salt is separated from the mixture via filtration.

7. Process for the preparation of enantiomerically enriched N-unprotected β-amino acid derivates, wherein a mixture of both enantiomers of an N-unprotected β-amino acid derivate is resolved through crystallisation of the salt of one enantiomer of the N-unprotected β-amino acid derivative and an enantiomerically pure N-protected α-amino acid.

8. Process according to claim 7, wherein the crystallisation is performed in a polar solvent.

9. Process according to claims 7 to 8, wherein the enantiomerically pure N-unprotected β-amino acid derivative is an ester or an amide.

10. Process according to claims 7 to 9, wherein the temperature during crystallisation is between -20°C and 30°C.

11. Process according to claims 7 to 10, wherein the crystallised salt is separated from the mixture via filtration.

## Patentansprüche

1. Verfahren zur Herstellung von enantiomerenangereicherten N-geschützten Aminosäuren, bei dem man ein Gemisch beider Enantiomere einer N-geschützten Aminosäure durch Kristallisation des Salzes aus einem Enantiomer der N-geschützten Aminosäure und eines enantiomerenreinen N-ungeschützten β-Aminosäurederivats spaltet.

2. Verfahren nach Anspruch 1, bei dem es sich bei der N-geschützten Aminosäure um eine N-geschützte α-Aminosäure handelt.

3. Verfahren nach Anspruch 1 und/oder 2, bei dem man die Kristallisation in einem polaren Lösungsmittel durchführt.

4. Verfahren nach einem oder mehreren der vorhergehenden Ansprüche, bei dem es sich bei dem enantiomerenreinen N-ungeschützten β-Aminosäurederivat um einen Ester oder ein Amid handelt.

5. Verfahren nach einem oder mehreren der vorhergehenden Ansprüche, bei dem die Temperatur während der Kristallisation zwischen -20°C und 30°C liegt.

6. Verfahren nach einem oder mehreren der vorhergehenden Ansprüche, bei dem man das kristallisierte Salz mittels Filtration aus dem Gemisch abtrennt.

7. Verfahren zur Herstellung von enantiomerenangereicherten N-ungeschützten β-Aminosäurederivaten, bei dem man ein Gemisch beider Enantiomere eines N-ungeschützten β-Aminosäurederivats durch Kristallisation des Salzes aus einem Enantiomer des N-ungeschützten β-Aminosäurederivats und einer enantiomerenreinen N-geschützten Aminosäure spaltet.

8. Verfahren nach Anspruch 7, bei dem man die Kristallisation in einem polaren Lösungsmittel durchführt.

9. Verfahren nach den Ansprüchen 7 bis 8, bei dem es sich bei dem enantiomerenreinen N-ungeschützten β-Aminosäurederivat um einen Ester oder ein Amid handelt.

10. Verfahren nach den Ansprüchen 7 bis 9, bei dem die Temperatur während der Kristallisation zwischen -20°C und 30°C liegt.

11. Verfahren nach den Ansprüchen 7 bis 10, bei dem man das kristallisierte Salz mittels Filtration aus dem Gemisch abtrennt.

**Revendications**

1. Procédé de préparation d’acides aminés N-protégés enrichis énantiomériquement, dans lequel un mélange des deux énantiomères d’un acide aminé N-protégé est dédoublé par cristallisation du sel d’un énantiomère de l’acide aminé N-protégé et d’un dérivé d’acide β-aminé N-non protégé énantiomériquement pur.

2. Procédé suivant la revendication 1, dans lequel l’acide aminé N-protégé est un acide α-aminé N-protégé.

3. Procédé suivant la revendication 1 et/ou 2, dans lequel la cristallisation est effectuée dans un solvant polaire.

4. Procédé suivant une ou plusieurs des revendications précédentes, dans lequel le dérivé d’acide β-aminé N-non protégé énantiomériquement pur est un ester ou un amide.

5. Procédé suivant une ou plusieurs des revendications précédentes, dans lequel la température pendant la cristallisation est comprise entre -20°C et 30°C.

6. Procédé suivant une ou plusieurs des revendications précédentes, dans lequel le sel cristallisé est séparé du mélange par filtration.

7. Procédé de préparation de dérivés d’acide β-aminé N-non protégé énantiomériquement enrichis, dans lequel un mélange des deux énantiomères d’un dérivé d’acide β-aminé N-non protégé est dédoublé par cristallisation du sel d’un énantiomère du dérivé d’acide β-aminé N-non protégé et d’un acide α-aminé N-protégé énantiomériquement pur.

8. Procédé suivant la revendication 7, dans lequel la cristallisation est effectuée dans un solvant polaire.

9. Procédé suivant les revendications 7 et 8, dans lequel le dérivé d’acide β-aminé N-non protégé énantiomériquement pur est un ester ou un amide.

10. Procédé suivant les revendications 7 à 9, dans lequel la température pendant la cristallisation est comprise entre

-20°C et 30°C.

**11.** Procédé suivant les revendications 7 à 10, dans lequel le sel cristallisé est séparé du mélange par filtration.